# FASCICULE DE BREVET EUROPEEN

(11) **EP 1 410 813 B1**
(45) Date de publication et mention de la délivrance du brevet: **30.05.2007**
(21) Numéro de dépôt: 03356153.1
(22) Date de dépôt: 15.10.2003
(51) Int. Cl.: A61M 1/16, A61M 1/34, B01D 61/30, A61L 2/02

(54) **Procédé et dispositif pour la préparation en ligne de liquide pour un appareil destiné au traitement extracorporel du sang**
Verfahren und Vorrichtung zur in-line Vorbereitung von Flüssigkeit für eine Vorrichtung zur Behandlung von Blut durch einen extrakorporalen Kreislauf
Process and device for the online preparation of liquid for an apparatus intended for extracorporeal blood treatment

(30) Priorité: 15.10.2002 FR 0212776
(43) Date de publication de la demande: 21.04.2004
(73) Titulaire: Gambro Lundia AB, 22 643 Lund (SE)
(72) Inventeur: Chevallet, Jacques, 69360 Serezin du Rhone (FR); Mercier, Guy, 69500 Bron (FR); Simard, Laurent, 69230 Saint Genis Laval (FR)
(74) Mandataire: Sutto, Luca

(56) Documents cités:
- EP-A- 0 462 606
- WO-A-02/32476
- US-A- 5 846 419
- US-B1- 6 187 207

## Description

### Domaine de l'invention

La présente invention concerne un procédé et un dispositif pour la préparation en ligne de liquide pour un appareil destiné au traitement extracorporel du sang, ainsi qu'un appareil pour le traitement du sang utilisant ledit procédé et ledit dispositif, comme décrit dans le document US-A-5 846 419.

En particulier, l'invention trouve une application dans la préparation du liquide destiné à être utilisé comme liquide d'infusion et/ou comme liquide de dialyse dans le traitement de substitution de la fonction rénale.

On sait que les patients affectés par une insuffisance rénale peuvent subir un traitement qui implique le prélèvement du sang du patient, le traitement du sang et la restitution du sang traité au même patient.

Dans ce but, le sang est normalement véhiculé dans un circuit extracorporel et est mis en circulation dans une première chambre d'une unité de traitement à membrane semi-perméable.

Plus précisément, dans le traitement de dialyse, il est prévu de faire circuler le sang dans une première chambre d'un dialyseur à deux chambres séparées par une membrane semi-perméable. Dans la deuxième chambre, on fait circuler un liquide de dialyse de composition chimique appropriée de manière à obtenir un transfert par diffusion des solutés à travers la membrane.

Dans le traitement d'hémofiltration, il est au contraire prévu d'extraire une fraction d'eau plasmatique à travers l'unité de traitement ou l'hémofiltre et simultanément de perfuser au patient un liquide de substitution pour compenser partiellement la quantité d'eau plasmatique prélevée à travers l'hémofiltre. Pendant le processus d'hémofiltration, il s'opère une migration des solutés par transport à travers la membrane semi-perméable de l'hémofiltre.

Enfin, l'hémodiafiltration est une combinaison des deux traitements qui viennent d'être décrits.

Le liquide de dialyse et le liquide de substitution sont des liquides présentant une composition chimique identique ou largement identique: ils sont essentiellement isotoniques et contiennent les électrolytes principaux du sang.

La production correcte de tels liquides et en particulier du liquide de substitution qui vient en contact direct avec le sang du patient constitue une problématique actuellement très préoccupante. En particulier, pour ne pas incommoder le patient, il s'est avéré important de garantir la production de liquides de substitution et éventuellement de liquides de dialyse qui soient stériles (c'est-à-dire exempts de bactéries ou de micro-organismes vivants) et non pyrogènes (c'est-à-dire exempts d'éléments pyrogènes dont l'introduction dans le sang est tenue responsable de dérangements tels que la fièvre, des frissonnements, des nausées, des réactions anaphylactiques).

Une solution technique actuellement adoptée par la présente demanderesse prévoit l'utilisation de trois stades de filtration: en premier lieu, on effectue une phase de filtration, par exemple d'eau potable normale, pour obtenir de l'eau à un degré élevé de pureté. Ensuite, l'eau ainsi obtenue est envoyée à la machine de dialyse dans laquelle sont prévus un deuxième et un troisième stade de filtration qui se succèdent. Plus en détail, le deuxième stade de filtration prévoit un filtre qui est remplacé mensuellement, tandis que le troisième stade de filtration prévoit une cartouche de petites dimensions et une ligne de conduits correspondants qui sont remplacés à la fin de chaque traitement.

Bien que la solution technique décrite ci-dessus opère de manière satisfaisante, elle implique l'utilisation de trois stades de filtration avec trois filtres différents l'un de l'autre, et elle oblige l'utilisateur à effectuer une action vigoureuse de désinfection à la fin de chaque traitement, en particulier à cause du fait que le deuxième filtre est réutilisé pendant une durée relativement longue. L'action de nettoyage et de désinfection entre deux séances successives doit en particulier être exécutée avec un soin et une attention extrême pour ne pas provoquer de dommages irréversibles à la membrane du deuxième filtre.

Par conséquent, un objet de la présente invention est de proposer un procédé et un dispositif pour la préparation en ligne de liquide, en mesure de demander un seul type de filtre et en même temps d'assurer un degré très élevé de stérilité du liquide produit, avec une simplification des procédures de désinfection par traitements successifs. Un autre objet de l'invention est un appareil pour le traitement extracorporel du sang qui utilise ledit procédé et ledit dispositif.

Ces objets, et d'autres, qui apparaîtront au cours de la description qui suit, sont essentiellement atteints avec :
- un procédé pour la préparation en ligne de liquide pour un appareil pour le traitement extracorporel du sang, lequel appareil comprend au moins un parcours de fluide 12 depuis une source de liquide jusqu'à une zone 4 d'injection dudit liquide dans un circuit extracorporel du sang et/ou dans un système cardio-vasculaire d'un patient et/ou dans une chambre d'un dialyseur, au moins un premier et un deuxième poste de filtration 16, 17 disposés le long dudit parcours de fluide, le deuxième poste de filtration travaillant en aval du premier poste de filtration, ledit procédé comprenant les phases consistant à:
   a) placer un filtre 18, 19 en correspondance à chaque poste 16,17 ;
   b) envoyer du liquide le long dudit parcours de fluide 12 à travers ledit premier et ledit deuxième postes de filtration 16, 17 vers ladite zone d'injection;
   c) enlever le filtre 18 qui travaille en correspondance au premier poste de filtration 16 ;
   d) placer en correspondance au premier poste 16 le filtre 19 qui travaille en correspondance au deuxième poste de filtration; et
   e) placer un nouveau filtre en correspondance au deuxième poste 19 et
- un dispositif de préparation en ligne de liquide, en particulier pour réaliser un procédé selon l'une des revendications précédentes, ledit dispositif comprenant:
   - un parcours de fluide 12 depuis une source de liquide jusqu'à une zone 4 d'injection dudit liquide dans un circuit extracorporel du sang et/ou dans le système cardio-vasculaire d'un patient et/ou dans une chambre d'un dialyseur ;
   - des moyens 15 pour déterminer un déplacement du liquide le long dudit parcours;
   - au moins un premier et un deuxième poste de filtration 16 et 17 disposés le long dudit parcours de fluide, le deuxième poste de filtration travaillant en aval dudit premier poste de filtration;
   - un filtre 18, 19 travaillant en correspondance à chaque poste de filtration;
   - un groupe de déplacement 26 agissant au moins sur le filtre qui travaille en correspondance audit deuxième poste pour déplacer ce filtre depuis ledit deuxième poste jusqu'audit premier poste.

### Brève description des dessins

D'autres caractéristiques et avantages apparaîtront mieux à la lecture de la description détaillée d'un mode de réalisation préféré mais non exclusif d'un procédé et d'un dispositif pour la préparation en ligne de liquide, ainsi que d'un appareil pour le traitement extracorporel du sang qui utilise ledit procédé et ledit dispositif selon la présente invention.

Cette description sera donnée ci-dessous en référence aux figures jointes, données à titre uniquement indicatif et non limitatif, dont :
- les figures 1A, 1B, 1C montrent schématiquement un appareil d'hémofiltration apte à utiliser le procédé et le dispositif selon l'invention ;

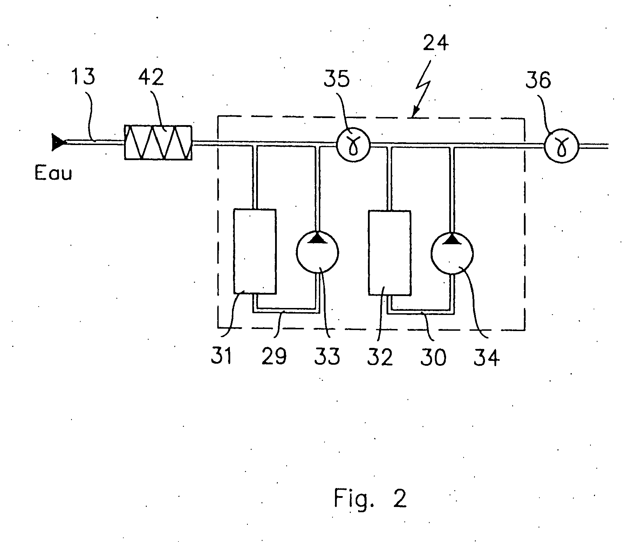

- la figure 2 montre une partie de l'appareil selon les figures 1A, 1B, 1C, relative au circuit de préparation du liquide.

### Description détaillée

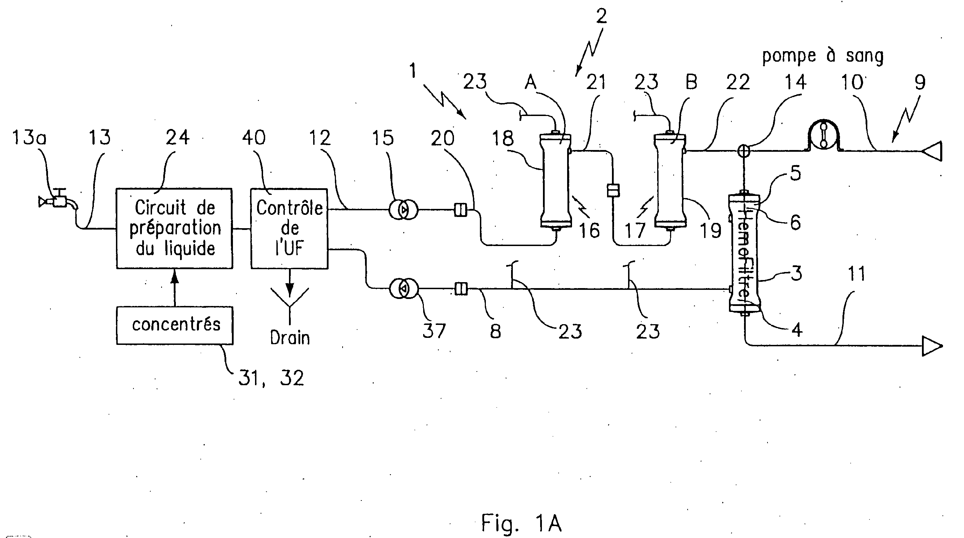

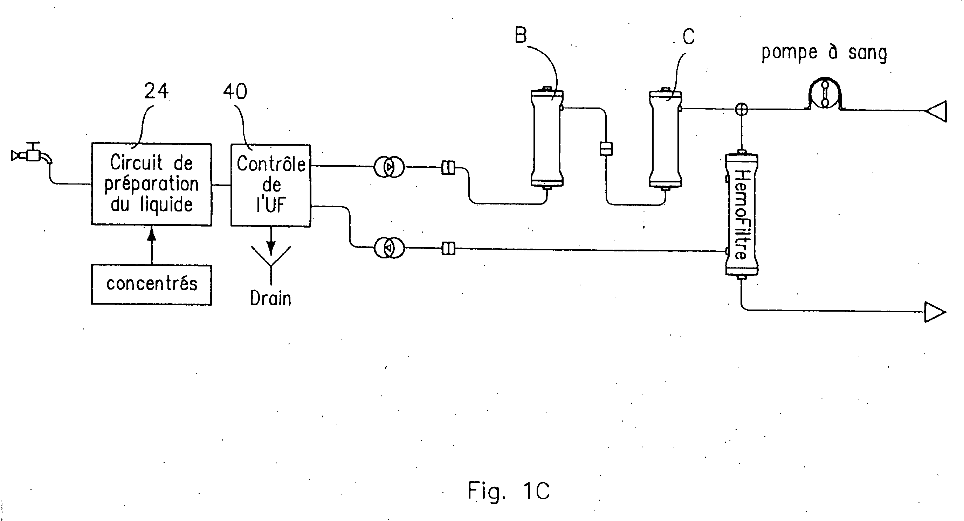

En référence aux figures annexées, on a désigné globalement par 1 un appareil pour le traitement extracorporel du sang. L'appareil 1 représenté dans les figures 1A et 1C est dans une configuration fonctionnelle qui lui permet d'effectuer un traitement d'hémofiltration. De toute manière, on notera que cette configuration est donnée purement à titre d'exemple et que la présente invention peut évidemment trouver une utilisation dans un traitement extracorporel quelconque du sang (par exemple hémodialyse, hémofiltration, hémodiafiltration) au cas où il se révélerait nécessaire ou commode de produire en ligne un liquide stérile. L'invention peut être utilisée pour produire un liquide de dialyse et/ou un liquide d'injection dans un circuit extracorporel du sang et/ou un liquide d'injection directe dans le système cardiovasculaire d'un patient. L'appareil 1 comprend au moins un dispositif de préparation en ligne de liquide, globalement désigné par 2 et encore décrit en détail plus loin, et au moins une unité 3 de traitement du sang. L'unité 3 présente une première chambre 4 et une deuxième chambre 5 qui sont séparées par au moins une membrane semi-perméable 6; la première chambre comprend une ouverture de sortie qui peut être raccordée à une ligne de décharge 8, tandis que la deuxième chambre 5 peut être raccordée à un circuit extracorporel du sang 9 présentant au moins une branche 10 qui prélève le sang du patient et au moins une branche 11 de restitution du sang au patient. Le dispositif de préparation en ligne de liquide 2 comprend un parcours de fluide 12 qui s'étend depuis une extrémité amont 13 qui peut être raccordée à une source d'eau courante 13a, jusqu'à une zone d'injection 14 de liquide dans le circuit extracorporel du sang et/ou directement dans le système cardio-vasculaire d'un patient. La ligne 12 peut être raccordée à la première chambre 4 dans le cas de préparation en ligne du liquide de dialyse. Dans l'exemple représenté, la zone d'injection 14 est placée en correspondance avec le circuit extracorporel 9 et en particulier sur la branche 10 de prélèvement du sang du patient, en amont de l'unité 3 (prédilution). De manière analogue, la zone d'injection peut être prévue en correspondance à la branche 11 (post-dilution) ou aux deux branches 10 et 11 (prédilution et post-dilution simultanées). Des moyens, qui comprennent par exemple une pompe 15, opèrent le long du parcours de fluide 12 pour amener un déplacement de liquide vers la zone d'injection 14. Le dispositif 2 comprend encore au moins un premier et un deuxième poste de filtration 16 et 17 disposés le long du parcours du fluide 12; comme le montre la figure jointe, le deuxième poste de filtration 17 est disposé en aval du premier poste de filtration 16 dans le sens d'avancement du liquide le long du parcours 12; un filtre respectif 18, 19 opère en correspondance avec chacun des postes de filtration. Des moyens de vanne classiques, non représentés, peuvent lorsqu'on le souhaite amener chaque filtre 18, 19 depuis une situation de fonctionnement dans laquelle le filtre est placé en communication d'écoulement avec le parcours de fluide jusque dans une situation de non fonctionnement dans laquelle le filtre est isolé hydrauliquement vis-à-vis du parcours de fluide, et inversement. Pour entrer plus dans les détails, on précisera que chacun des filtres 18, 19 (identiques entre eux sur le plan structurel) comprennent au moins une première chambre et une deuxième chambre séparées par une membrane de filtration, au moins une ouverture d'accès à ladite première chambre et au moins une ouverture de sortie de ladite deuxième chambre. A son tour, le parcours de fluide 12 présente une première partie 20 apte à raccorder la source de liquide à la première chambre du filtre 18 qui travaille dans ledit premier poste, une deuxième partie 21 apte à raccorder la deuxième chambre du filtre 18 qui travaille dans ledit premier poste à la première chambre du filtre 19 qui travaille dans ledit deuxième poste, et une troisième partie 22 apte à raccorder la deuxième chambre du filtre 19 qui travaille dans ledit deuxième poste à ladite zone d'injection 14. De cette manière, le liquide qui parvient à la zone d'injection 14 a traversé nécessairement tant le filtre 18 que le filtre 19.

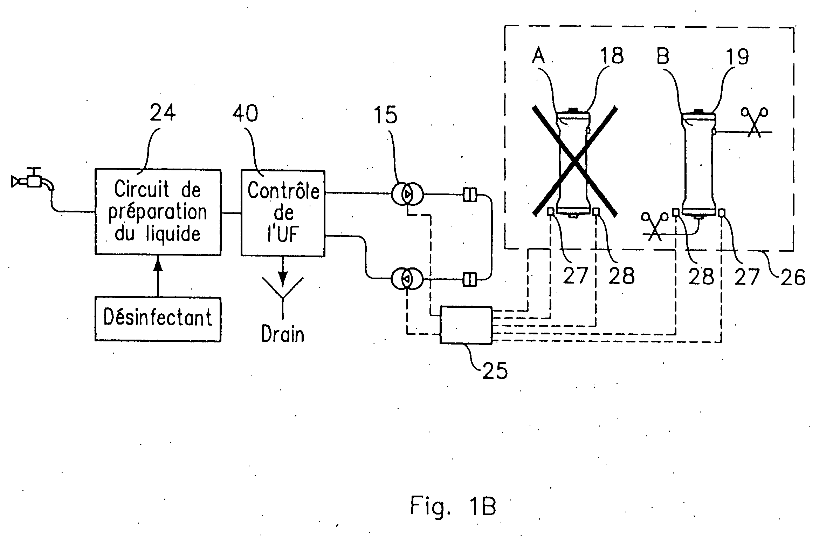

Chaque filtre comprend également une ouverture de sortie en correspondance à ladite première chambre, pour effectuer un déplacement du fluide dans ladite première chambre du filtre, tangentielle par rapport à la membrane de filtration. Dans ce but, les ouvertures de sorties des filtres peuvent être raccordées à la ligne de décharge 8 ou à des décharges indépendantes, au moyen de conduits 23 sur lesquels agissent des organes de régulation de l'écoulement, contrôlés par une unité de contrôle 25 (figure 1B). Les organes de régulation de l'écoulement peuvent par exemple comprendre une vanne et/ou une pompe qui travaille sur chacun desdits conduits 23. L'unité de contrôle 25 peut commander les organes de régulation pour réaliser un lavage tangentiel de la première chambre, en continu ou par intervalles de temps successifs. On notera qu'en correspondance à chaque poste, on peut également prévoir une ligne de recirculation (non représentée) apte à renvoyer vers l'ouverture d'entrée au moins une partie du liquide évacué par l'ouverture de sortie de ladite première chambre.

Un groupe de déplacement 26 agissant sur le filtre 18 et 19 est capable d'amener le filtre travaillant dans le premier poste vers un poste de décharge et de déplacer vers ledit premier poste le filtre présent dans le deuxième poste. L'unité de commande 25 coordonne l'actionnement du groupe de déplacement par l'activation des moyens 15. En pratique, pendant une session de préparation de liquide, qui correspond à une phase de traitement du patient, l'unité 25 commande les moyens 15 en provoquant un écoulement de liquide de portée appropriée le long du parcours 12; lorsque la session de préparation de liquide est terminée, l'unité 25 procède à l'actionnement du groupe de déplacement 26 pour amener dans le poste de sortie le filtre qui travaille dans le premier poste et pour amener dans le premier poste le filtre présent dans le deuxième poste.

On notera que des capteurs 27 en mesure de relever la présence d'un filtre dans chaque poste et d'envoyer un signal correspondant à l'unité de commande 25 opèrent en correspondance au premier et au deuxième poste; chaque filtre peut être également doté d'un code d'identification qui peut être lu par des détecteurs 28 appropriés (des détecteurs par exemple optiques, électriques, magnétiques ou, électromagnétiques, capables en tous cas de recevoir une information portée par le filtre) qui travaillent en correspondance avec chaque poste et apte à émettre un signal d'identification respectif à l'unité de commande 25.

L'unité 25 peut être programmée pour permettre la préparation en ligne de liquide et les capteurs 27 indiquent que les filtres respectifs sont effectivement installés dans les postes. De plus, grâce à la possibilité d'identifier l'identité de chaque filtre, l'unité 25 peut être programmée de manière à permettre la préparation en ligne de liquide uniquement si dans le premier poste est utilisé un filtre (filtre B dans la figure 1B) qui n'a été utilisé que pour une seule session précédente (ou un filtre neuf - filtre A dans la figure 1A) et si dans le deuxième poste est utilisé un filtre neuf (filtre B dans la figure 1A ; filtre C dans la figure 1C). Au cas où les conditions mentionnées ci-dessus ne seraient pas respectées, l'unité 25 peut être programmée pour émettre un signal d'alarme et/ou pour interrompre l'attribution du traitement à des parties de l'appareil 1.

Enfin, en relation au mode de réalisation spécifique représenté, le dispositif 2 comprend en outre un circuit de préparation 24 ayant un organe de réchauffement 42 qui opère en aval de l'extrémité 13, et au moins deux canalisations de dérivation 29, 30 qui se suivent le long du parcours 2. Une cartouche ou un conteneur de concentrés 31, 32 (par exemple un seul) ainsi qu'une pompe 33, 34 opèrent sur chaque canalisation de dérivation. Le long du parcours 2, en aval de la zone concernée par chaque canalisation sont placés des capteurs de concentration ou de conductivité 35, 36 en mesure d'envoyer des signaux correspondants à l'unité 25. En comparant les valeurs relevées par les capteurs à des valeurs de consigne, l'unité 25 peut réguler de manière appropriée la portée de la pompe vers chaque canalisation auxiliaire. Le long du tube de décharge 8 peut être prévue une pompe de décharge 37 en aval de laquelle opère un débitmètre. Egalement en correspondance au parcours 12, en amont de la pompe 15, est prévu un autre débitmètre. Les débitmètres sont partie d'un circuit de contrôle de l'ultrafiltration 40. Grâce aux signaux provenant des débitmètres et grâce à un contrôle approprié de toutes les pompes décrites plus haut, il est possible de gérer de manière appropriée l'ultrafiltration et donc la perte de poids du patient.

En utilisation, le dispositif 2 décrit est en mesure de mettre en oeuvre un procédé qui fait également partie de la présente invention et qui comprend les phases décrites ci-dessous. En activant pour la première fois le dispositif 2, on procède à l'installation un filtre, nouveau et stérile, en correspondance à chaque poste; ensuite, en même temps que l'on exécute le traitement extracorporel, les moyens 15 sont activés pour envoyer du liquide le long dudit parcours de fluide 12, à travers les postes de filtration et les filtres 18, 19, vers la zone d'injection 14. Lorsque le traitement ou la traversée à duré un certain temps (éventuellement programmable), on procède à l'enlèvement du filtre qui travaille en correspondance au premier poste de filtration et à l'isolement du filtre présent dans ledit deuxième poste vis-à-vis du parcours du fluide 12; enfin, on effectue une procédure de nettoyage et de désinfection du parcours de fluide 12 et, si cela s'impose, des différents conduits de l'appareil 1 sans que les filtres 18, 19, soient concernés dans cette opération (figure 1B). On notera qu'il n'est pas nécessaire de désinfecter le filtre 19 présent dans le deuxième poste. Lorsque l'on doit effectuer un nouveau traitement, on procède au placement dans le premier poste du filtre présent en correspondance au deuxième poste de filtration et au placement dans le deuxième poste d'un nouveau filtre stérile; ensuite, le dispositif est prêt pour une nouvelle session, à la fin de laquelle on procède à l'enlèvement du filtre du premier poste et à son remplacement par celui présent dans le deuxième poste, comme on l'a déjà décrit plus haut. Si les phases d'enlèvement et de remplacement des filtres devaient être effectuées périodiquement et non à la fin de chaque traitement, avant de procéder à l'enlèvement des filtres des postes respectifs, l'écoulement de liquide le long du parcours 2 sera en tout cas interrompu.

L'invention offre d'importants avantages.

En premier lieu, la stérilité du liquide en aval du deuxième poste de filtration est garantie puisque le filtre en aval est toujours neuf et stérile. On remarquera en outre que le filtre le plus affecté par l'éventuelle présence de particules indésirables dans le filtre est le filtre amont qui remplit sa fonction pendant un seul cycle et qui est donc remplacé sans risque que des bactéries ou des agents pyrogènes soient disséminés suite à une désinfection inefficace. En outre, puisqu'il n'y a pas d'actions de désinfection, les filtres usagés ne peuvent être endommagés ni entraîner une propagation des agents indésirables. Le tout est obtenu avec une simplicité extrême, puisque des filtres utilisés sont identiques, avec des avantages supplémentaires en termes de gestion.

## Revendications

1. Procédé pour la préparation en ligne de liquide pour un appareil pour le traitement extracorporel du sang, lequel appareil comprend au moins un parcours de fluide (12) depuis une source de liquide jusqu'à une zone (14) d'injection dudit liquide dans un circuit extracorporel du sang et/ou dans un système cardio-vasculaire d'un patient et/ou dans une chambre d'un dialyseur, au moins un premier et un deuxième poste de filtration (16 et 17) disposés le long dudit parcours de fluide, le deuxième poste de filtration travaillant en aval du premier poste de filtration,
ledit procédé comprenant les phases consistant à:
a) placer un filtre (18, 19) en correspondance à chaque poste (16, 17);
b) envoyer du liquide le long dudit parcours de fluide (12) à travers ledit premier et ledit deuxième postes de filtration (16 et 17) vers ladite zone d'injection;
c) enlever le filtre (18) qui travaille en correspondance au premier poste de filtration (16);
d) placer en correspondance au premier poste (16) le filtre (19) qui travaille en correspondance au deuxième poste de filtration; et
e) placer un nouveau filtre en correspondance au deuxième poste (19).

2. Procédé selon la revendication 1, **caractérisé en ce que** les phases a), b), c), d), e) se suivent l'une l'autre dans le temps.

3. Procédé selon la revendication 1, **caractérisé en ce qu'**après ladite phase de placement d'un nouveau filtre, il est prévu de répéter successivement les phases b), c), d), e) les unes après les autres.

4. Procédé selon la revendication 1, **caractérisé en ce que** chaque phase (b) pendant laquelle le fluide est envoyé vers ladite zone d'injection (14) correspond à l'administration d'un traitement extracorporel à un patient par ledit appareil, les phases c), d), e) étant effectuées une fois que le traitement est terminé.

5. Procédé selon la revendication 1, **caractérisé en ce que** chaque phase b) pendant laquelle le fluide est envoyé vers ladite zone d'injection (14) correspond à un intervalle de temps prédéterminé.

6. Procédé selon la revendication 1, **caractérisé en ce que** chaque phase b) pendant laquelle le fluide est envoyé vers ladite zone d'injection (14) correspond à un intervalle de temps programmable par un utilisateur.

7. Procédé selon la revendication 2, **caractérisé en ce qu'**entre lesdites phases b) et c) est prévue une phase d'interruption de l'écoulement de liquide le long dudit parcours (12).

8. Procédé selon la revendication 2, **caractérisé en ce qu'**entre lesdites phases c) et d) sont prévues les sous-phases consistant à:
- isoler les filtres (19) dudit deuxième poste (17) par rapport audit passage de fluide;
- effectuer une opération de désinfection dudit parcours de fluide (12) sans agir sur le filtre (19) présent dans ledit deuxième poste (17).

9. Procédé selon la revendication 1, **caractérisé en ce que** les filtres (18, 19) travaillant dans ledit premier et dans ledit deuxième poste sont structurellement identiques l'un à l'autre.

10. Procédé selon la revendication 1, **caractérisé en ce qu'**en correspondance audit deuxième poste est placé un filtre (19) stérile, pour définir une barrière capable de garantir en utilisation la stérilité du liquide en aval dudit deuxième poste.

11. Procédé selon la revendication 1, **caractérisé en ce que** chacun desdits filtres (18, 19) comprend au moins une première et une deuxième chambre (18a et 18b, 19a et 19b) séparées entre elles par une membrane de filtration (18c, 19c), au moins une ouverture d'accès à ladite première chambre et au moins une ouverture de sortie de ladite deuxième chambre.

12. Procédé selon la revendication 11, **caractérisé en ce que** ledit parcours de fluide comprend:
- une première partie (20) agencée pour raccorder la source de liquide à la première chambre du filtre qui travaille dans ledit premier poste,
- une deuxième partie (21) agencée pour raccorder la deuxième chambre du filtre travaillant dans ledit premier poste à la première chambre de filtre travaillant dans ledit deuxième poste,
- une troisième partie (22) agencée pour raccorder la deuxième chambre du filtre travaillant dans ledit deuxième poste avec ladite zone d'injection.

13. Procédé selon la revendication 11, **caractérisé en ce que** chaque filtre (18, 19) comprend également une ouverture de sortie en correspondance à ladite première chambre, pour effectuer un déplacement de fluide dans ladite première chambre du filtre tangentiellement par rapport à la membrane de filtration (18c, 19c).

14. Procédé selon la revendication 13, **caractérisé en ce que** ladite phase de déplacement tangentiel est effectuée périodiquement.

15. Procédé selon la revendication 13, **caractérisé en ce que** ladite phase de déplacement tangentiel est effectuée en continu.

16. Dispositif de préparation en ligne de liquide, en particulier pour réaliser un procédé selon l'une des revendications précédentes, ledit dispositif comprenant:
- un parcours de fluide (12) depuis une source de liquide jusqu'à une zone(14) d'injection dudit liquide dans un circuit extracorporel du sang et/ou dans le système cardio-vasculaire d'un patient et/ou dans une chambre d'un dialyseur ;
- des moyens (15) pour déterminer un déplacement du liquide le long dudit parcours;
- au moins un premier et un deuxième poste de filtration (16 et 17) disposés le long dudit parcours de fluide, le deuxième poste de filtration travaillant en aval dudit premier poste de filtration;
- un filtre (18, 19) travaillant en correspondance à chaque poste de filtration;
- un groupe de déplacement (26) agissant au moins sur le filtre qui travaille en correspondance audit deuxième poste pour déplacer ce filtre depuis ledit deuxième poste jusqu'audit premier poste.

17. Dispositif selon la revendication 16, **caractérisé en ce que** ledit groupe de déplacement (26) agit en outre sur le filtre (18) qui travaille dans ledit premier poste pour déterminer son enlèvement dudit premier poste.

18. Dispositif selon la revendication 16, **caractérisé en ce qu'**il comprend une unité de commande (25) qui agit sur ledit groupe de déplacement et capable d'exécuter les opérations ci-dessous:
- activer lesdits moyens (15) pour déterminer un déplacement du liquide le long dudit parcours pendant une session de préparation de liquide;
- lorsque la session de préparation est terminée, commander ledit groupe de déplacement (26) pour qu'il apporte dans le poste de décharge le filtre travaillant dans le premier poste et pour amener dans le premier poste le filtre présent dans le deuxième poste.

19. Dispositif selon la revendication 16, **caractérisé en ce que** le filtre présent dans ledit deuxième poste est stérile.

20. Dispositif selon la revendication 18, **caractérisé en ce qu'**il comprend des capteurs (27) associés audit premier et audit deuxième poste pour relever la présence d'un filtre et envoyer un signal correspondant à l'unité de commande.

21. Dispositif selon la revendication 18, **caractérisé en ce qu'**il comprend des détecteurs (28) associés audit premier et audit deuxième poste pour relever l'identité d'un filtre placé dans chacun desdits postes et envoyer un signal correspondant à l'unité de commande.

22. Dispositif selon la revendication 20, **caractérisé en ce que** l'unité de commande est programmée pour permettre la préparation en ligne de liquide si les capteurs (27) indiquent la présence desdits filtres dans les postes respectifs.

23. Dispositif selon la revendication 21, **caractérisé en ce que** l'unité de commande est programmée pour permettre la préparation en ligne de liquide si les détecteurs (28) indiquent que dans le premier poste est utilisé un filtre qui n'a été utilisé que pour une seule session précédente ou un filtre neuf et que dans le deuxième poste est utilisé un filtre neuf.

24. Dispositif selon la revendication 16, **caractérisé en ce que** chaque filtre présente au moins une première et une deuxième chambre (18a et 18b, 19a et 19b) séparées entre elles par une membrane de filtration (18c, 19c), au moins une ouverture d'accès à ladite première chambre et au moins une ouverture de sortie de ladite deuxième chambre.

25. Dispositif selon la revendication 18, **caractérisé en ce que** ledit parcours de fluide comprend:
- une première partie (20) agencée pour relier la source de liquide à la première chambre du filtre qui travaille dans ledit premier poste,
- une deuxième partie (21) agencée pour raccorder la deuxième chambre du filtre travaillant dans ledit premier poste à la première chambre du filtre travaillant dans ledit deuxième poste, et
- une troisième partie (22) agencée pour raccorder la deuxième chambre du filtre travaillant dans ledit deuxième poste avec ladite zone d'injection.

26. Dispositif selon la revendication 23, **caractérisé en ce que** chaque filtre comprend également une ouverture de sortie en correspondance à ladite première chambre pour effectuer, périodiquement ou en continu, un déplacement du fluide dans la première chambre du filtre, tangentiel par rapport à la membrane de filtration.

27. Appareil pour le traitement extracorporel du sang, comprenant:
- au moins un dispositif (2) de préparation en ligne de liquide selon l'une des revendications précédentes, et
- au moins une unité de traitement du sang (3) présentant une première chambre et une deuxième chambre séparées par au moins une membrane semi-perméable, la première chambre présentant au moins une ouverture de sortie qui peut être raccordée à une ligne de décharge et la deuxième chambre pouvant être raccordée à un circuit extracorporel du sang (9).

28. Appareil selon la revendication 27, **caractérisé en ce que** ladite zone d'injection (14) dudit liquide est raccordée au circuit extracorporel du sang en amont et/ou en aval de l'unité de traitement (3).

29. Appareil selon la revendication 27, **caractérisé en ce que** ladite zone d'injection (14) dudit liquide est raccordée à ladite première chambre de l'unité (3).

## Claims

1. A method for preparing on-line liquid for an apparatus for extracorporeal blood treatment, which apparatus comprises at least one fluid path (12) from a liquid source to an injection area (14) of said liquid into an extracorporeal blood circuit and/or into a patient's cardio-vascular system and/or into a compartment of a dialyzer, at least a first and second filtration point (16 and 17) arranged along said fluid path, the second filtration point operating downstream from the first filtration point,
said method comprising the following steps:
a) arranging a filter (18, 19) on each point (16, 17);
b) sending liquid along said fluid path (12) through said first and said second filtration points (16 and 17) to said injection area;
c) removing the filter (18) operating on the first filtration point (16);
d) arranging on the first point (16) the filter (19) operating on the second filtration point; and
e) arranging a new filter on the second point (19).

2. The method according to claim 1, **characterized in that** the steps a), b), c), d), e) follow one another in time.

3. The method according to claim 1, **characterized in that** after said step of arranging a new filter, it is envisaged to repeat said steps b), c), d), e) one after the other.

4. The method according to claim 1, **characterized in that** every step (b) in which the fluid is sent to said injection area (14) corresponds to the administration of an extracorporeal treatment to a patient by means of said apparatus, the steps c), d), e) being carried out after the treatment is over.

5. The method according to claim 1, **characterized in that** every step b) in which the fluid is sent to said injection area (14) corresponds to a pre-established time interval.

6. The method according to claim 1, **characterized in that** every step b) in which the fluid is sent to said injection area (14) corresponds to a time interval that can be programmed by a user.

7. The method according to claim 2, **characterized in that** between said steps b) and c) it is provided for a step of interrupting the flow of liquid along said path (12).

8. The method according to claim 2, **characterized in that** between said steps c) and d) it is provided for the sub-steps of:
- isolating the filters (19) of said second point (17) with respect to said fluid passage;
- disinfecting said fluid path (12) without acting upon the filter (19) present in said second point (17).

9. The method according to claim 1, **characterized in that** the filters (18, 19) operating in said first and in said second point are structurally identical to one another.

10. The method according to claim 1, **characterized in that** on said second point a sterile filter (19) is arranged, so as to define a barrier that is able to ensure during operation the sterility of the liquid downstream from said second point.

11. The method according to claim 1, **characterized in that** each of said filters (18, 19) comprises at least a first and a second compartment (18a and 18b, 19a and 19b) separated from one another by means of a filtration membrane (18c, 19c), at least one inlet opening to said first compartment and at least one outlet opening from said second compartment.

12. The method according to claim 11, **characterized in that** said fluid path comprises:
- a first portion (20) designed to connect the liquid source to the first compartment of the filter operating in said first point,
- a second portion (21) designed to connect the second compartment of the filter operating in said first point to the first compartment of the filter operating in said second point,
- a third portion (22) designed to connect the second compartment of the filter operating in said second point to said injection area.

13. The method according to claim 11, **characterized in that** each filter (18, 19) further comprises an outlet opening on said first compartment, so as to carry out a displacement of fluid in said first compartment of the filter tangentially to the filtration membrane (18c, 19c).

14. The method according to claim 13, **characterized in that** said step of tangential displacement is carried out periodically.

15. The method according to claim 13, **characterized in that** said step of tangential displacement is carried out continuously.

16. A device for preparing on-line liquid, in particular for carrying out a method according to one of the preceding claims, said device comprising:
- a fluid path (12) from a liquid source to an injection area (14) of said liquid into an extracorporeal blood circuit and/or into a patient's cardio-vascular system and/or into a compartment of a dialyzer;
- means (15) for detecting a displacement of the liquid along said path;
- at least a first and second filtration point (16 and 17) arranged along said fluid path, the second filtration point operating downstream from said first filtration point;
- a filter (18, 19) operating on each filtration point;
- a displacement group (26) acting at least upon the filter operating on said second point for displacing said filter from said second point to said first point.

17. The device according to claim 16, **characterized in that** said displacement group (26) further acts upon the filter (18) operating in said first point so as to detect its removal from said first point.

18. The device according to claim 16, **characterized in that** it comprises a control unit (25) acting upon said displacement group and able to execute the following operations:
- activating said means (15) for detecting a displacement of the liquid along said path during a session of liquid preparation;
- when the preparation session is over, controlling said displacement group (26) so that it leads to said outflow point the filter operating in the first point and for leading to the first point the filter present in the second point.

19. The device according to claim 16, **characterized in that** the filter present in said second point is sterile.

20. The device according to claim 18, **characterized in that** it comprises sensors (27) associated to said first and to said second point for detecting the presence of a filter and sending a corresponding signal to the control unit.

21. The device according to claim 18, **characterized in that** it comprises detectors (28) associated to said first and to said second point for detecting the identity of a filter placed in each of said points and sending a corresponding signal to the control unit.

22. The device according to claim 20, **characterized in that** the control unit is programmed to enable the on-line preparation of liquid if the sensors (27) indicate the presence of said filters in the respective points.

23. The device according to claim 21, **characterized in that** the control unit is programmed to enable the on-line preparation of liquid if the detectors (28) indicate that in the first point a filter that has only been used for one preceding session or a new filter is used, and that in the second point a new filter is used.

24. The device according to claim 16, **characterized in that** each filter comprises at least a first and a second compartment (18a and 18b, 19a and 19b) separated from one another by means of a filtration membrane (18c, 19c), at least one inlet opening to said first compartment and at least one outlet opening from said second compartment.

25. The device according to claim 18, **characterized in that** said fluid path comprises:
- a first portion (20) designed to connect the liquid source to the first compartment of the filter operating in said first point,
- a second portion (21) designed to connect the second compartment of the filter operating in said first point to the first compartment of the filter operating in said second point, and
- a third portion (22) designed to connect the second compartment of the filter operating in said second point to said injection area.

26. The device according to claim 23, **characterized in that** each filter further comprises an outlet opening on said first compartment so as to carry out, either periodically or continuously, a displacement of fluid in the first compartment of the filter tangentially to the filtration membrane.

27. An apparatus for extracorporeal blood treatment, comprising:
- at least one device (2) for preparing on-line liquid according to one of the preceding claims, and
- at least one blood treatment unit (3) having a first compartment and a second compartment separated by means of at least one semipermeable membrane, the first compartment having at least one outlet opening that can be connected to an outflow line and the second compartment being able to be connected to an extracorporeal blood circuit (9).

28. The apparatus according to claim 27, **characterized in that** said injection area (14) of said liquid is connected to the extracorporeal blood circuit upstream and/or downstream from the treatment unit (3).

29. The apparatus according to claim 27, **characterized in that** said injection area (14) of said liquid is connected to said first compartment of the unit (3).

## Patentansprüche

1. Verfahren zur Online-Vorbereitung von Flüssigkeit für ein Gerät zur extrakorporalen Blutbehandlung, wobei das Gerät mindestens einen Flüssigkeitsweg (12) von einer Flüssigkeitsquelle bis zum einem Injektionsbereich (14) der Flüssigkeit in einen extrakorporalen Blutkreislauf und/oder in ein Herz-Gefäß-System eines Patienten und/oder in einen Abteil eines Dialysegeräts, mindestens eine erste und eine zweite Filtrationsstelle (16 und 17), die entlang dem Flüssigkeitsweg angeordnet sind, umfasst, wobei die zweite Filtrationsstelle abwärts von der ersten Filtrationsstelle arbeitet,
wobei das Verfahren die folgenden Schritte umfasst:
a) Anordnung eines Filters (18, 19) an jeder Stelle (16, 17);
b) Zuführung der Flüssigkeit entlang dem Flüssigkeitsweg (12) durch die erste und die zweite Filtrationsstellen (16 und 17) zum Injektionsbereich;
c) Entfernung des Filters (18), der an der ersten Filtrationsstelle (16) arbeitet;
d) Anordnung an der ersten Stelle (16) des Filters (19), der an der zweiten Filtrationsstelle arbeitet; und
e) Anordnung eines neuen Filters an der zweiten Stelle (19).

2. Verfahren nach Anspruch 1, **dadurch gekennzeichnet, daß** die Schritte a), b), c), d), e) sind zeitlich aufeinanderfolgend.

3. Verfahren nach Anspruch 1, **dadurch gekennzeichnet, daß** nach dem Schritt der Anordnung eines neuen Filters es vorgesehen ist, die Schritte b), c), d) e) aufeinanderfolgend zu wiederholen.

4. Verfahren nach Anspruch 1, **dadurch gekennzeichnet, daß** jeder Schritt (b), bei dem die Flüssigkeit zum Injektionsbereich (14) zugeführt wird, der Verabreichung einer extrakorporalen Behandlung zu einem Patienten durch das Gerät entspricht, wobei die Schritte c), d), e) nach der Beendigung der Behandlung durchgeführt werden.

5. Verfahren nach Anspruch 1, **dadurch gekennzeichnet, daß** jeder Schritt b), bei dem die Flüssigkeit zum Injektionsbereich (14) zugeführt wird, einem vorgegebenen Zeitintervall entspricht.

6. Verfahren nach Anspruch 1, **dadurch gekennzeichnet, daß** jeder Schritt b), bei dem die Flüssigkeit zum Injektionsbereich zugeführt wird, einem von einem Benutzer programmierbaren Zeitintervall entspricht.

7. Verfahren nach Anspruch 2, **dadurch gekennzeichnet, daß** zwischen den Schritten b) und c) ein Schritt vorgesehen ist, bei dem der Flüssigkeitsfluss entlang dem Weg (12) unterbrochen wird.

8. Verfahren nach Anspruch 2, **dadurch gekennzeichnet, daß** zwischen den Schritten c) und d) die untergeordneten Schritten vorgesehen sind, umfassend:
- die Isolierung der Filter (19) der zweiten Stelle (17) gegenüber dem Flüssigkeitsdurchgang;
- die Durchführung einer Desinfektion des Flüssigkeitswegs (12), ohne auf den in der zweiten Stelle (17) vorliegenden Filter (19) zu wirken.

9. Verfahren nach Anspruch 1, **dadurch gekennzeichnet, daß** die in der ersten und in der zweiten Stelle arbeitenden Filter (18,19) identisch aufgebaut sind.

10. Verfahren nach Anspruch 1, **dadurch gekennzeichnet, daß** an der zweiten Stelle ein steriler Filter (19) angeordnet ist, um eine Barriere zu definieren, die bei der Verwendung die Sterilität der Flüssigkeit abwärts von der zweiten Stelle sicherstellen kann.

11. Verfahren nach Anspruch 1, **dadurch gekennzeichnet, daß** jeder der Filter (18, 19) mindestens einen ersten und einen zweiten Abteil (18a und 18b, 19a und 19b), die durch eine Filtrationsmembran (18c, 19c) voneinander getrennt sind, mindestens eine Zugangsöffnung zum ersten Abteil und mindestens eine Ausgangsöffnung vom zweiten Abteil umfasst.

12. Verfahren nach Anspruch 11, **dadurch gekennzeichnet, daß** der Flüssigkeitsweg:
- einen ersten Abschnitt (20) zur Verbindung der Flüssigkeitsquelle mit dem ersten Abteil des in der ersten Stelle arbeitenden Filters,
- einen zweiten Abschnitt (21) zur Verbindung des zweiten Abteils des in der ersten Stelle arbeitenden Filters mit dem ersten Abteil des in der zweiten Stelle arbeitenden Filters,
- einen dritten Abschnitt (22) zur Verbindung des zweiten Abteils des in der zweiten Stelle arbeitenden Filters mit dem Injektionsbereich, umfasst.

13. Verfahren nach Anspruch 11, **dadurch gekennzeichnet, daß** jeder Filter (18, 19) ebenfalls eine Ausgangsöffnung am ersten Abteil umfasst, um eine Flüssigkeitsverschiebung im ersten Abteil des Filter tangential zur Filtrationsmembran (18c, 19c) durchzuführen.

14. Verfahren nach Anspruch 13, **dadurch gekennzeichnet, daß** der Schritt der tangentialen Verschiebung periodisch durchgeführt wird.

15. Verfahren nach Anspruch 13, **dadurch gekennzeichnet, daß** der Schritt der tangentialen Verschiebung ständig durchgeführt wird.

16. Vorrichtung zur Online-Vorbereitung von Flüssigkeit, insbesondere zur Durchführung eines Verfahrens nach einem der vorstehenden Ansprüche, wobei die Vorrichtung:
- einen Flüssigkeitsweg (12) von einer Flüssigkeitsquelle bis zum einem Injektionsbereich (14) der Flüssigkeit in einen extrakorporalen Blutkreislauf und/oder in ein Herz-Gefäß-System eines Patienten und/oder in einen Abteil eines Dialysegeräts;
- Mittel (15) zur Erfassung einer Verschiebung der Flüssigkeit entlang dem Weg;
- mindestens eine erste und eine zweite Filtrationsstelle (16 und 17), die entlang dem Flüssigkeitsweg angeordnet sind, wobei die zweite Filtrationsstelle abwärts von der ersten Filtrationsstelle arbeitet;
- einen Filter (18, 19), der an jeder Filtrationsstelle arbeitet;
- eine Verschiebungsanordnung (26), die mindestens auf den an der zweiten Stelle arbeitenden Filter wirkt, um den Filter von der zweiten Stelle bis zur ersten Stelle zu verschieben, umfasst.

17. Vorrichtung nach Anspruch 16, **dadurch gekennzeichnet, daß** die Verschiebungsanordnung (26) auch auf den in der ersten Stelle arbeitenden Filter (18) arbeitet, um dessen Entfernung von der ersten Stelle zu erfassen.

18. Vorrichtung nach Anspruch 16, **dadurch gekennzeichnet, daß** sie eine Steuerung (25) umfasst, die auf die Verschiebungsanordnung wirkt und die folgenden Operationen ausführen kann:
- Betätigung der Mittel (15) zur Erfassung einer Flüssigkeitsverschiebung entlang dem Weg bei einer Flüssigkeitsvorbereitungssitzung;
- wenn die Vorbereitungssitzung beendet wird, Ansteuerung der Verschiebungsanordnung (26), so daß sie in die Ablaufstelle den in der ersten Stelle arbeitenden Filter verschiebt und in die erste Stelle den in der zweiten Stelle vorliegenden Filter führt.

19. Vorrichtung nach Anspruch 16, **dadurch gekennzeichnet, daß** der in der zweiten Stelle vorliegende Filter steril ist.

20. Vorrichtung nach Anspruch 18, **dadurch gekennzeichnet, daß** sie Fühler (27) umfasst, die mit der ersten und mit der zweiten Stelle verbunden sind, um die Anwesenheit eines Filters zu erfassen und ein entsprechendes Signal zur Steuerung zu senden.

21. Vorrichtung nach Anspruch 18, **dadurch gekennzeichnet, daß** sie Sensoren (28) umfasst, die mit der ersten und mit der zweiten Stelle verbunden sind, um die Identität eines in jeder der Stellen angeordneten Filters zu erfassen und ein entsprechendes Signal zur Steuerung zu senden.

22. Vorrichtung nach Anspruch 20, **dadurch gekennzeichnet, daß** die Steuerung programmiert ist, um die Online-Vorbereitung von Flüssigkeit zu ermöglichen, wenn die Fühler (27) die Anwesenheit der Filter in den jeweiligen Stellen anzeigen.

23. Vorrichtung nach Anspruch 21, **dadurch gekennzeichnet, daß** die Steuerung programmiert ist, um die Online-Vorbereitung von Flüssigkeit zu ermöglichen, wenn die Sensoren (28) anzeigen, daß in der ersten Stelle ein Filter, der nur für eine vorstehende Sitzung verwendet wurde, oder ein neuer Filter verwendet wird, und daß in der zweiten Stelle ein neuer Filter verwendet wird.

24. Vorrichtung nach Anspruch 16, **dadurch gekennzeichnet, daß** jeder Filter mindestens einen ersten und einen zweiten Abteil (18a und 18b, 19a und 19b), die durch eine Filtrationsmembran (18c, 19c) voneinander getrennt sind, mindestens eine Zugangsöffnung zum ersten Abteil und mindestens eine Ausgangsöffnung vom zweiten Abteil umfasst.

25. Vorrichtung nach Anspruch 18, **dadurch gekennzeichnet, daß** der Flüssigkeitsweg:
- einen ersten Abschnitt (20) zur Verbindung der Flüssigkeitsquelle mit dem ersten Abteil des in der ersten Stelle arbeitenden Filters,
- einen zweiten Abschnitt (21) zur Verbindung des zweiten Abteils des in der ersten Stelle arbeitenden Filters mit dem ersten Abteil des in der zweiten Stelle arbeitenden Filters, und
- einen dritten Abschnitt (22) zur Verbindung des zweiten Abteils des in der zweiten Stelle arbeitenden Filters mit dem Injektionsbereich,
umfasst.

26. Vorrichtung nach Anspruch 23, **dadurch gekennzeichnet, daß** jeder Filter ebenfalls eine Ausgangsöffnung am ersten Abteil umfasst, um eine Flüssigkeitsverschiebung im ersten Abteil des Filter tangential zur Filtrationsmembran periodisch oder ständig durchzuführen.

27. Gerät zur extrakorporalen Blutbehandlung, umfassend:
- mindestens eine Vorrichtung (2) zur Online-Vorbereitung von Flüssigkeit nach einem der vorstehenden Ansprüche, und
- mindestens eine Blutbehandlungseinheit (3) mit einem ersten Abteil und einem zweiten Abteil, die durch mindestens eine semipermeable Membran voneinander getrennt sind, wobei der erste Abteil mindestens eine Ausgangsöffnung aufweist, die mit einer Ablaufleitung verbunden werden kann, und wobei der zweite Abteil mit einem extrakorporalen Blutkreislauf (9) verbunden werden kann.

28. Gerät nach Anspruch 27, **dadurch gekennzeichnet, daß** der Injektionsbereich (14) der Flüssigkeit mit dem extrakorporalen Blutkreislauf aufwärts und/oder abwärts von der Behandlungseinheit (3) verbunden ist.

29. Gerät nach Anspruch 27, **dadurch gekennzeichnet, daß** der Injektionsbereich (14) der Flüssigkeit mit dem ersten Abteil der Einheit (3) verbunden ist.
